# EUROPEAN PATENT SPECIFICATION

(11) **EP 0 910 402 B1**
(45) Date of publication and mention of the grant of the patent: **08.08.2007**
(21) Application number: 97927018.8
(22) Date of filing: 19.06.1997
(51) Int. Cl.: A61K 38/28, A61K 47/26

(54) **INSULIN PREPARATIONS CONTAINING MANNITOL**
INSULINPREPARATION MIT MAnnitol
PREPARATIONS A BASE D'INSULINE CONTENANT MANNITOL

(30) Priority: 20.06.1996 DK 68496; 27.08.1996 DK 89996
(43) Date of publication of application: 28.04.1999
(73) Proprietor: NOVO NORDISK A/S, 2880 Bagsvaerd (DK)
(72) Inventor: KIMER, Lone, Loegstrup, DK-3520 Farum (DK); BALSCHMIDT, Per, DK-3060 Espergaerde (DK); JENSEN, Steen, DK-2791 Dragoer (DK)
(86) International application number: PCT/DK1997/000267
(87) International publication number: WO 1997/048413

(56) References cited:
- WO-A-95/00550
- US-A- 4 439 181
- CENTRE FOR BIOTECHNOLOGY, Vol. 70, VINITA GUPTA et al., "Effect of Solvent Additives on the Thermal Stability of Insulin", pages 209-212.

## Description

### Introduction

The present invention relates to aqueous insulin preparations comprising human insulin or an analogue or derivative thereof, which preparations have superior physical stability. The invention furthermore relates to parenteral formulations comprising such insulin preparations and to a method for improving the physical stability of insulin preparations.

### Background of the invention

### Field of the invention

Diabetes is a general term for disorders in man having excessive urine excretion as in diabetes mellitus and diabetes insipidus. Diabetes mellitus is a metabolic disorder in which the ability to utilize glucose is more or less completely lost. About 2% of all people suffer from diabetes.

Since the introduction of insulin in the 1920's, continuos strides have been made to improve the treatment of diabetes mellitus. To help avoid extreme glycemia levels, diabetic patients often practice multiple injection therapy, whereby insulin is administered with each meal.

In the treatment of diabetes mellitus, many varieties of insulin preparations have been suggested and used, such as regular insulin, Semilente^{®} insulin, isophane insulin, insulin zinc suspensions, protamine zinc insulin, and Ultralente^{®} insulin. As diabetic patients are treated with insulin for several decades, there is a major need for safe and life quality improving insulin preparations. Some of the commercial available insulin preparations are characterized by a fast onset of action and other preparations have a relatively slow onset but show a more or less prolonged action. Fast acting insulin preparations are usually solutions of insulin, while retarded acting insulin preparations can be suspensions containing insulin in crystalline and/or amorphous form precipitated by addition of zinc salts alone or by addition of protamine or by a combination of both. In addition, some patients are using preparations having both a fast onset of action and a more prolonged action. Such a preparation may be an insulin solution wherein protamine insulin crystals are suspended. Some patients do themselves prepare the final preparation by mixing an insulin solution with a suspension preparation in the ratio desired by the patient in question.

Normally, insulin preparations are administered by subcutaneous injection. What is important for the patient, is the action profile of the insulin preparation which is the action of insulin on the glucose metabolism as a function of the time from the injection. In this profile, inter alia, the time for the onset, the maximum value and the total duration of action are important. A variety of insulin preparations with different action profiles are desired and requested by the patients. One patient may, on the same day, use insulin preparations with very different action profiles. The action profile requested is, for example, depending on the time of the day and the amount and composition of any meal eaten by the patient.

Equally important, however, is the physical stability of the insulin preparations due to the abundant use of pen-like injection devices such as devices which contain Penfill^{®} cartridges, in which an insulin preparation is stored until the entire cartridge is empty. This may last for at least 1 to 2 weeks for devices containing 1.5-3.0 ml cartridges.

### Description of the background art

The first stable neutral insulin suspension was developed by Scott and Fischer (J. Pharmacol. Exp. Ther. 58 (1936), 78) who discovered that the presence of a surplus of protamine and a zinc salt (2 µg zinc per IU (international Unit) insulin) could stabilize the protamine insulin preparation, described by Hagedorn et al.: J.Am.Med.Assn. 106 (1936), 177 - 180.

Protamine Zinc Insulin made according to the United States or European Pharmacopoeias contains amorphous protamine zinc insulin as well as crystalline Protamine Zinc Insulin. Freshly prepared protamine zinc insulin contains mainly amorphous precipitate which will partly be transformed into crystalline particles upon storage, leading to a more protracted effect.

A completely crystalline protamine zinc insulin modification designated NPH insulin or Isophane Insulin was developed by Krayenbühl and Rosenberg (see Rep. Steno Mem. Hosp. Nord. Insulinlab. 1 (1946), 60; and Danish patent No. 64,708). They found that insulin and protamine brought together in isophane proportions at a neutral pH value in the presence of a small amount of zinc and phenol, or phenol derivatives or, preferably m-cresol, will form an amorphous precipitate which upon standing is gradually but completely transformed into oblong tetragonal crystals limited at the ends by pyramidal faces. Insulin and salmon protamine co-crystallize in a weight ratio corresponding to about 0.09 mg protamine sulphate per mg insulin. Zinc in an amount of at least 0.15 µg per IU and a phenolic in a concentration higher than 0.1% is necessary for the preparation of the tetragonal crystals.

In the early days, this kind of crystals were prepared using, porcine and bovine insulin from natural sources, but from the eighties also human insulin, made by genetic engineering or by semisynthesis, is used.

Human insulin consists of two polypeptide chains, the so-called A and B chains which contain 21 and 30 amino acids, respectively. The A and B chains are interconnected by two cystine disulphide bridges. Insulin from most other species has a similar construction, but may not contain the same amino acids at the positions corresponding in the chains as in human insulin.

The development of the process known as genetic engineering has made it possible easily to prepare a great variety of insulin compounds being analogous to human insulin. In these insulin analogues, one or more of the amino acids have been substituted with other amino acids which can be coded for by the nucleotide sequences. As human insulin, as explained above, contains 51 amino acid residues, it is obvious that a large number of insulin analogues is possible and, in fact, a great variety of analogues with interesting properties have been prepared. In human insulin solutions with a concentration of interest for injection preparations, the insulin molecule is present in associated form as a hexamer (Brange et al. Diabetes Care 13, (1990), 923 - 954). After subcutaneous injection, it is believed that the rate of absorption by the blood stream is dependent of the size of the molecule, and it has been found that insulin analogues with amino acid substitutions which counteract or inhibit this hexamer formation have an unusual fast onset of action (Brange et al.: Ibid). This is of great therapeutic value for the diabetic patient. In the crystals of the prolonged acting protamine insulin preparations, the insulin is also found to be hexameric (Balschmidt et al.: Acta Chryst. B47, (1991), 975 - 986).

Pharmaceutical preparations which are based on analogues of human insulin are e.g. known from the following documents:

WO 95/00550 is concerned with pharmaceutical preparations based on insulin crystals comprising Asp^{B28} insulin and protamine, which display rapid onset and prolonged activity when administered in vivo. The crystals may furthermore contains zinc ions and phenol and/or m-cresol. Glycerol is added to the preparations as isotonicity agent.

In US 5 461 031 various parenteral pharmaceutical formulations, which comprise a rapid-acting monomeric insulin analogue, zinc, protamine and a phenolic derivative, is disclosed. The formulations furthermore contain glycerol which acts as an isotonicity agent

US 5 474 978 discloses a rapid acting parenteral formulation comprising a human insulin analogue hexamer complex consisting of six monomeric insulin analogues, zinc ions and at least three molecules of a phenolic derivative. The preferred isotonicity agent is glycerol.

Unfortunately, insulin tends to form insoluble and biologically inactive fibrils by non-covalent polymerization (c.f. e.g. Jens Brange, Galenics of Insulin, Springer-Verlag, 1987 and references therein). Fibril formation is promoted by elevated temperatures, e.g. above 30°C, and concomitant movements. This fibrillation process, which is very difficult to avoid, poses an upper limit on the period for which the insulin preparation can be stored and, hence, on the cartridge volume of Penfills^{®}.

The effect of sugars and polyols on thermal stability of bovine insulin has also been investigated (V. Gupta, R. Bhat Centre for Biotechnology, Vol 70, pg 209-212)

Since the formation of fibrils generally requires a monomerization of insulin, insulin analogues which more reluctantly form di- and hexamers, result in preparations of less physical stability due to fibrillation.

Thus, it is an object of the present invention to provide an insulin preparation comprising human insulin analogue with improved physical stability.

According to the invention this object has been accomplished by An aqueous insulin preparation comprising:
a) dissolved insulin analogue and crystals comprising insulin analogue and protamine,
b) 100 to 400 mM of mannitol,
c) 5 to 40 mM of a chloride,
d) a physiologically tolerated buffer, preferably a phosphate buffer,
wherein the concentration of insulin analogue is 60 to 3000 nmol/ml; the weight ratio between dissolved and crystalline insulin analogue is 30:70 to 70:30; the crystals comprises zinc and a phenolic compound; and the analogue of human insulin is one wherein position B28 is Asp, Lys, Leu, Val or Ala and position B29 is Lys or Pro, or des(B28-B30), des(B27) or des(B30) human insulin

### Brief description of the drawing

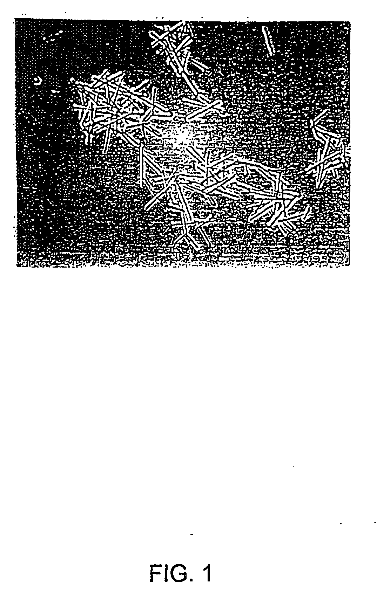

Fig 1 is a photomicrograph (x1000 magnification) of a formulation of the invention comprising Asp^{B28} human insulin-protamine crystals and mannitol.

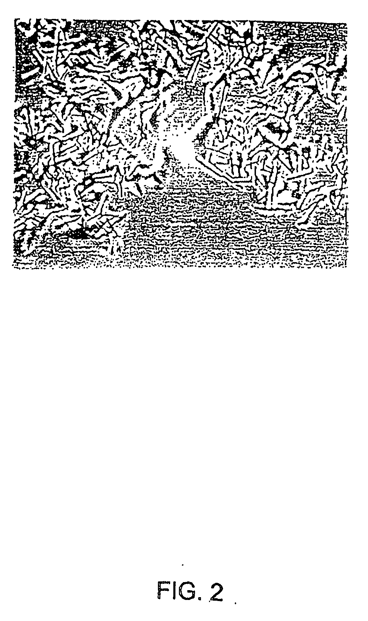

Fig 2 is a photomicrograph (x1000 magnification) of a formulation of the invention comprising human insulin-protamine crystals and mannitol.

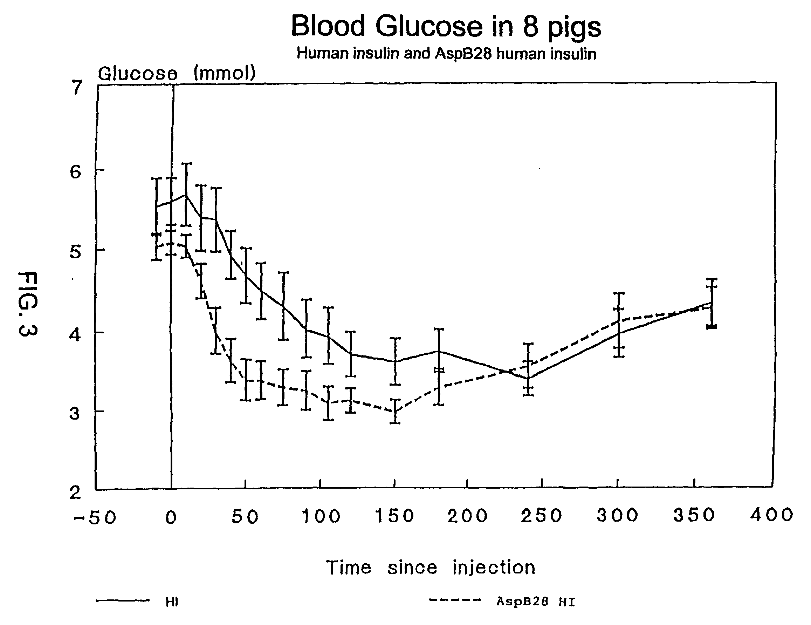

Fig 3 is a graphical representation of the profile of action of a preparation of the invention containing both dissolved and crystalline ASp^{B28} human insulin and a preparation containing both dissolved and crystalline human insulin. Both preparations furthermore contain mannitol. The graph is the blood glucose response after injection in pigs. The figure demonstrate that the rapid onset of action is preserved for the highly stable Asp^{B28} human insulin preparation.

### Description of the invention

### Definitions

By "analogue of human insulin" as used herein is meant human insulin in which one or more amino acids have been deleted and/or replaced by other amino acids, including non-codeable amino acids, or human insulin comprising additional amino acids, i.e. more than 51 amino acids.

In the present context the term "water-soluble" corresponds to a solubility in water of at least about 10 mmol/l, preferably at least 50 mmol/l, at a temperature of 20°C.

The terms carbohydrate, reduced carbohydrate, monosaccharide, disaccharide, and ester and ether derivatives of such compounds are used in accordance with the teaching of K. A. Jensen in "Grundrids af den organiske kemi, Almen Kemi III, 1. Ed., Jul. Gjellerups forlag, 1969, pp. 299-316.

In the present context the expression "non-reducing carbohydrate" denotes a carbohydrate which essentially is incapable of reacting with the amino groups of insulin in the preparations of the invention to form glycated insulin. This definition includes carbohydrates in which the carbonyl group(s) has/have been inactivated or blocked, e.g. by anhydride formation or derivatization.

In one aspect, the present invention relates to An aqueous insulin preparation comprising:
a) dissolved insulin analogue and crystals comprising insulin analogue and protamine,
b) 100 to 400 mM of mannitol,
c) 5 to 40 mM of a chloride,
d) a physiologically tolerated buffer, preferably a phosphate buffer,
wherein the concentration of insulin analogue is 60 to 3000 nmol/ml; the weight ratio between dissolved and crystalline insulin analogue is 30:70 to 70:30; the crystals comprises zinc and a phenolic compound; and the analogue of human insulin is one wherein position B28 is Asp, Lys, Leu, Val or Ala and position B29 is Lys or Pro, or des(B28-B30), des(B27) or des(B30) human insulin

### Preferred embodiments

Since insulin preparations comprising fast-acting analogues of human insulin generally show a rather low physical stability, the present invention is particularly advantageous in connection with preparations comprising such analogues. Thus, the insulin preparation according to the invention preferably comprises one or more fast-acting analogues of human insulin, in particular analogues wherein position B28 is Asp, Lys, Leu, Val or Ala and position B29 is Lys or Pro; or des(B28-B30), des(B27) or des(B30) human insulin. The insulin analogue is preferably selected from analogues of human insulin wherein position B28 is Asp or Lys, and position B29 is Lys or Pro. The most preferred analogues are Asp^{B28} human insulin or LYS^{B28}Pro^{B29} human insulin.

In a preferred embodiment the insulin preparation comprises both dissolved and precipitated, preferably crystalline, insulin analogue in a weight ratio of 30:70 to 70:30.

In this embodiment of the invention, the insulin preparation advantageously comprises crystals comprising: insulin analogue and protamine, and zinc and a phenolic compound, such as phenol, m-cresol or a mixture thereof. The amount of protamine in the crystals preferably corresponds to 0.20 to 0.40 mg protamine base/100 IU insulin or insulin analogue. The ratio of protamine to insulin in the crystals more preferably corresponds to the isophane ratio. Zinc is preferably present in an amount of 10 to 40 µg Zn/100 IU insulin, more preferably 15 to 35 µg Zn/100 IU insulin. Phenol and m-cresol, respectively, is preferably present in an amount corresponding to 0 to 4 mg/ml. However, a mixture of 1.4 to 2.0 mg/ml m-cresol and 0.6 to 2.0 mg/ml phenol is most preferred.

A preferred insulin preparation of the invention comprises:
a) 60 to 3000 nmol/ml, preferably 240 to 1200 nmol/mL of human insulin or insulin analogue and/or insulin derivative;
b) mannitol, in a concentration of 100 to 400 mM, preferably 150 to 250 mM, more preferably 180 to 230 mM;
c) sodium chloride, in a concentration of 0 to 100 mM, preferably 5 to 40 mM, more preferably 5 to 20 mM; and
d) a physiologically tolerated buffer, preferably a phosphate buffer such as disodiumphosphatedihydrate in an amount of 1 to 4 mg/ml.

The preparation of the invention may furthermore contain one or more compounds commonly used as isotonicity agents, such as glycerol.

The pH value of the insulin preparation is preferably in the range 7.0 to 7.8. A method for preparing an insulin preparation containing both dissolved and precipitated insulin analogue is provided but not claimed, which method comprises the following steps:
a) providing an acidic solution comprising an analogue of human insulin, zinc, and a sub-isophanic amount of protamine;
b) providing an alkaline solution comprising a substance which acts as a buffer at physiological pH;
   wherein at least one of the above solutions further comprises a phenolic compound;
c) mixing the acidic and alkaline solutions and, optionally, adjusting the pH to a value in the range of 6.5 to 8.0, preferably 7.0 to 7.8; and
d) leaving the resulting suspension for precipitation.

By this method an insulin preparation containing both dissolved and precipitated insulin analogue can be obtained in a very simple manner. Furthermore, the precipitate of the resulting suspension usually consists of md-shaped crystals, which are advantageous in so-called PreMix insulin preparations.

The weight ratio of insulin analogue to protamine in the solution of step a) is preferably selected so as to obtain in the final product a weight ratio of dissolved to precipitated insulin analogue in the range of 1:99 to 99:1, preferably 20:80 to 80:20, more preferably 30:70 to 70:30. More specifically, the solution of step a) preferably comprises 120 to 6000 nmol/ml of insulin analogue and 0.01 to 5.0 mg/ml of protamine.

The solution of step a) further comprises zinc, preferably in an amount corresponding to 10 - 40 ug zinc/100 IU insulin, more preferably 15 - 35 ug zinc/I 00 TU insulin.

In a preferred embodiment, solution a) and/or solution b) comprises chloride, preferably sodium chloride, in an amount corresponding to 0 to 100 mM, preferably 5 to 40 mM, more preferably 5 to 20 mM, in the final product.

The pH of the acidic solution of step a) is preferably below 5, more preferably in the range of 2 to 3.5.

The insulin analogue is preferably human insulin wherein position B28 is Asp, Lys, Leu, Val or Ala and position B29 is Lys or Pro; or des(B28-B30), des(B27) or des(B30) human insulin, more preferably Asp^{B28} human insulin or Lys^{B28}Pro^{B29} human insulin, still more preferably Asp^{B28} human insulin.

The phenolic compound used in the solution of step a) and/or step b) is preferably phenol, m-cresol or a mixture thereof.

Advantageously, the solution of step a) and/or step b) further comprises a water-soluble reduced or non-reducing carbohydrate containing at least 4 carbon atoms in the main carbohydrate structure, or a water-soluble non-reducing ester and/or ether derivative of a carbohydrate or reduced carbohydrate containing at least 4 carbon atoms in the main carbohydrate structure, or mixtures thereof.

Said carbohydrate or carbohydrate derivative preferably contains from 5 to 18 carbon atoms in the main carbohydrate structure.

In a particular preferred embodiment, the solution of step a) and/or step b) comprises mannitol, sorbitol, xylitol, inositol, trehalose, sucrose or any mixture thereof, preferably mannitol and/or sorbitol, more preferably mannitol.

The buffer substance employed in the alkaline solution of step b) is preferably a physiologically tolerated buffer, preferably a phosphate buffer, more preferably disodium phosphate dihydrate.

The precipitated insulin analogue is preferably in the form of crystals comprising insulin analogue and protamine.

The suspension obtained in step d) is preferably left at a temperature in the range of 5°C to 40°C, more preferably 20°C to 36°C, still more preferably 30°C to 34°C, for precipitation.

This invention is further illustrated by the following examples

### EXAMPLE I

### Preparation 1

An insulin preparation containing both dissolved and crystalline Asp^{B28} human insulin was prepared in the following way:

### Solution A:

A solution of Asp^{B28} human insulin at 200 IU/ml concentration was prepared by dissolving 76.5 mg of Asp^{B28} human insulin in water by adding to it 326 µl 0.2 N hydrochloric Acid and 163 µl zinc chloride solution (0.4 mg/ml). Then 6.35 mg protamine sulphate in solution was added to the insulin solution while mixing, and a mixture consisting of 17.2 mg m-cresol, 15 mg phenol and 455 mg mannitol was added to this solution while mixing. The pH of the resulting clear solution was measured to pH=2.6-2.9, and water ad 10 ml was added. The solution was equilibrated at 28-32°C.

### Solution B:

25 mg disodium phosphate dihydrate was dissolved in Water for Injection. 17.2 mg m-cresol, 15 mg phenol and 455 mg mannitol was added during mixing. The pH of the resulting clear solution was measured to 9, and water ad 10 ml was added. The solution was equilibrated at 28-32°C.

### Mixing of solution A and B:

Solution B was added to solution A, and the pH was readjusted to 7.30. The resulting suspension was left at 30°C for 6 days for crystallisation.

In the resulting preparation, the weight ratio of precipitated to dissolved insulin was 70:30.

The preparation was introduced into 1.5 ml Penfill^{®} cartridges.

### EXAMPLE II

### Preparation 2

An insulin preparation containing both dissolved and crystalline Asp^{B28} human insulin was prepared in the following way:

### i) Crystalline fraction.

### Solution A:

A solution of Asp^{B28} human insulin at 200 IU/ml concentration was prepared by dissolving 190.3 mg of Asp^{B28} human insulin in water by adding to it 813 µl 0.2 N hydrochloric acid and 410 µl zinc chloride solution (0.4 mg/ml). Then 16.1 mg protamine sulphate in solution was added to the insulin solution while mixing, and a mixture consisting of 43.0 mg m-cresol, 37.5 mg phenol and 909 mg mannitol, and 14.6 mg sodium chloride was added to this solution while mixing. The pH of the resulting clear solution was measured to pH=2.6-2.9, and water ad 22 ml was added. The solution was equilibrated at 32°C.

### Solution B:

62.4 mg disodium phosphate dihydrate was dissolved in water. 43.0 mg m-cresol, 37.5 mg phenol, 909 mg mannitol, and 14.6 mg sodium chloride was added during mixing. The pH of the resulting clear solution was measured to 9, and water ad 22 ml was added. The solution was equilibrated at 32°C.

### Mixing of solution A and B:

Solution B was added to solution A, and the pH was readjusted to 7.30, and water ad 50 ml was added. The resulting suspension was left at 32°C for 4 days for crystallization.

In the resulting preparation, the weight ratio of precipitated to dissolved insulin was 70:30.

The preparation was introduced into 1.5 ml Penfill^{®} cartridges.

### EXAMPLES III-VI

### Preparations 3 to 6

Insulin preparations containing both dissolved and crystalline Asp^{B28} human insulin was prepared as described in Example II, except that the amount of mannitol used in Solution A and B was 818 mg, 1005 mg, 1047 mg and 1137 mg, respectively.

The preparations were introduced into 1.5 ml Penfill^{®} cartridges.

### EXAMPLE VII

### Preparation 7

An insulin preparation containing both dissolved and crystalline Lys^{B28}Pro^{B29} human insulin was prepared in the following way:

### i) Crystalline fraction.

### Solution A:

A solution of Lys^{B28}Pro^{B29} human insulin at 200 IU/ml concentration was prepared by suspending 69.7 mg of Lys^{B28}Pro^{B29} human insulin in water. A mixture consisting of 16.0 mg m-cresol, 6.5 mg phenol, 364 mg mannitol and 25.1 mg disodium diphosphate dihydrate was added to this solution while mixing. Then 50 µl zinc chloride solution (10 mg/ml) was added. The pH of the resulting clear solution was readjusted to 7.40, and water ad 10 ml was added. The solution was equilibrated at 15°C.

### Solution B:

A solution of protamine sulphate was prepared by dissolving 7.61 mg protamine sulphate and 25.1 mg disodium phosphate dihydrate in water. 16.0 mg m-cresol, 6.5 mg phenol and 364 mg mannitol was added during mixing. This solution was added to the protamine sulphate solution while mixing. The pH of the resulting clear solution was readjusted to 7.40, and water ad 10 ml was added. The solution was equilibrated at 15°C.

### Mixing of solution A and B:

Solution B was added to solution A, and the pH was readjusted to 7.30. The resulting suspension was left at 15°C for 3 days for crystallization.

### ii) Dissolved fraction

A solution of Lys^{B28}Pro^{B29} human insulin was prepared by dissolving 34.9 mg Lys^{B28}Pro^{B29} human insulin in water by adding to it 33 µl N hydrochloric acid and 25 µl zinc chloride solution (10 mg/ml). Then a mixture consisting of 26.1 mg sodium phosphate dihydrate, 6.5 mg phenol, 16.0 mg m-cresol and 364 mg mannitol was added to the insulin solution while mixing. The pH of the resulting clear solution was measured to pH = 7.3, and water ad 10 ml was added.

6 ml of the dissolved fraction was added to 14 ml of the crystalline fraction, and the pH was adjusted to 7.30.

In the resulting preparation, the weight ratio of precipitated to dissolved insulin was 70:30.

The preparation was introduced into 1.5 ml Penfill^{®} cartridges.

### EXAMPLE VIII

### Preparation 8

An insulin preparation containing both dissolved and crystalline human insulin was prepared in the following way:

### i) Crystalline fraction

### Solution A:

A solution of human insulin was prepared by dissolving 69.7 mg human insulin in water by adding to it 65 µl 1 N hydrochloric acid and 26 µl zinc chloride solution (10 mg/ml). Then 6.0 mg protamine sulphate in solution was added to the insulin solution while mixing, and a mixture consisting of 15.0 mg phenol and 17.2 mg m-cresol was added to the solution while mixing. The pH of the resulting clear solution was measured to pH = 2.7 - 3.2, and water ad 10 ml was added.

### Solution B:

24.9 mg sodium phosphate dihydrate was dissolved in water. 15 mg phenol, 17.2 mg m-cresol, 728 mg mannitol and 11.7 mg sodium chloride was added during mixing. The pH of the resulting clear solution was adjusted to pH = 9, and water ad 10 ml was added.

Solution B was added to solution A, and the pH was adjusted to 7.30. The resulting suspension was left at 22-24 °C until the next day.

### ii) Dissolved fraction

A solution of human insulin was prepared by dissolving 34.9 mg human insulin in water by adding to it 33 µl 1 N hydrochloric acid and 13 µl zinc chloride solution (10 mg/ml). Then a mixture consisting of 12.5 mg sodium phosphate dihydrate, 15 mg phenol, 17.2 mg m-cresol, 364 mg mannitol and 5.8 mg sodium chloride was added to the insulin solution while mixing. The pH of the resulting clear solution was measured to pH = 7.3, and water ad 10 ml was added.

6 ml of the dissolved fraction was added to 14 ml of the crystalline fraction, and the pH was adjusted to 7.30.

In the resulting preparation, the weight ratio of precipitated to dissolved insulin was 70:30.

The preparation was introduced into 1.5 ml Penfill^{®} cartridges.

### EXAMPLE IX

### Preparation 9

An insulin preparation containing both dissolved and crystalline Asp^{B28} human insulin was prepared in the following way:

### Solution A:

A solution of Asp^{B28} human insulin at 200 IU/ml concentration was prepared by dissolving 189.9 mg of Asp^{B28} human insulin in water by adding to it 163 µl 1 N hydrochloric acid and 163.5 µl zinc chloride solution (10 mg/ml). Then 11.5 mg protamine sulphate in solution was added to the insulin solution while mixing, and a mixture consisting of 44.3 mg m-cresol, 38.6 mg phenol and 1048 mg mannitol', and 7.3 mg sodium chloride was added to this solution while mixing. The pH of the resulting clear solution was measured to pH=2.6-2.9, and water ad 25 ml was added. The solution was equilibrated at 22-24°C.

### Solution B:

62.3 mg disodium phosphate dihydrate was dissolved in water. 44.3 mg m-cresol, 38.6 mg phenol, 1048 mg mannitol, and 7.3 mg sodium chloride was added during mixing. The pH of the resulting clear solution was measured to 9, and water ad 25 ml was added. The solution was equilibrated at 22-24°C.

### Mixing of solution A and B:

Solution B was added to solution A, and the pH was readjusted to 7.30. The resulting suspension was left at 32°C for 2 days for crystallisation.

In the resulting preparation, the weight ratio of precipitated to dissolved insulin was 50:50.

The preparation was introduced into 1.5 ml Penfill^{®} cartridges.

### EXAMPLE X (Comparative)

### Preparation 10

An insulin preparation containing both dissolved and crystalline Asp^{B28} human insulin was prepared in the following way:

### Solution A:

A solution of Asp^{B28} human insulin at 200 IU/ml concentration was prepared by dissolving 76.5 mg of Asp^{B28} human insulin in water by adding to it 326 µl 0.2 N hydrochloric acid and 163 µl zinc chloride solution (0.4 mg/ml). Then 6.35 mg protamine sulphate in solution was added to the insulin solution while mixing, and a mixture consisting of 17.2 mg m-cresol, 15.0 mg phenol and 160 mg glycerol was added to this solution while mixing. The pH of the resulting clear solution was measured to pH=2.6-2.9, and water ad 10 ml was added. The solution was equilibrated at 28-32°C.

### Solution B:

25 mg disodium phosphate dihydrate was dissolved in Water for Injection. 17.2 mg m-cresol, 15.0 mg phenol and 160 mg glycerol was added during mixing. The pH of the resulting clear solution was measured to 9, and water ad 10 ml was added. The solution was equilibrated at 28-32°C.

Solution B was added to solution A, and the pH was readjusted to 7.30. The resulting suspension was left at 28-32°C for 2 days for crystallization.

In the resulting preparation, the weight ratio of precipitated to dissolved insulin was 70:30.

The preparation was introduced into 1.5 ml Penfill^{®} cartridges.

### EXAMPLE XI

### PHYSICAL STRESS TESTS

5 samples of each insulin preparation were introduced into Penfill^{®} cartridges and subjected to the following physical stress test:

The Penfill^{®} cartridges were fixed to a rotator which were placed in an incubator, and rotated 360° for four hours per day with a frequency of 30 rotations per minute and at a constant temperature of 37°C±2°C. The Penfill^{®} cartridges were stored at a temperature of 37°C±2°C, when they were not rotated.

The Penfill^{®} cartridges were inspected macroscopically 5 times a week and changes in the appearance of the formulation were noted according to following principles:21
i) Cartridges containing a white suspension resuspendable on agitation and free of lumps and granules were classified as "not fibrillated".
ii) Cartridges containing a suspension with lumps and/or granules not resuspendable on agitation and/or depositing on the cartridge wall were assumed "fibrillated". This was confirmed by addition of 6 µl 6N HCl to the cartridge: Fibrillated cartridges are not visually clear after addition of acid.

Rotation of the cartridges was continued until all samples were fibrillated.

The results are summarized in the following Table I

**TABLE I**

| Preparation No. | Composition | Average No. of days to fibrillation | First day of fibrillation |
|---|---|---|---|
| 1 | Asp^{B28} insulin 30/70 | 20 | 20 |
| | 7 mM phosphate | | |
| | 250 mM mannitol | | |
| 2 | Asp^{B28} insulin 30/70 | 23 | 23 |
| | 7 mM phosphate | | |
| | 200 mM mannitol | | |
| | 10 mM sodium chloride | | |
| 3 | Asp^{B28} insulin 30/70 | 21 | 17 |
| | 7 mM phosphate | | |
| | 180 mM mannitol | | |
| | 10 mM sodium chloride | | |
| 4 | Asp^{B28} insulin 30/70 | 19 | 17 |
| | 7 mM phosphate | | |
| | 220 mM mannitol | | |
| | 10 mM sodium chloride | | |
| 5 | Asp^{B28} insulin 30/70 | 21 | 18 |
| | 7 mM phosphate | | |
| | 230 mM mannitol | | |
| | 10 mM sodium chloride | | |
| 6 | Asp^{B28} insulin 30/70 | 22 | 22 |
| | 7 mM phosphate | | |
| | 250 mM mannitol | | |
| | 10 mM sodium chloride | | |
| 9 | Asp^{B28} insulin 50/50 | >30 | >30 |
| | 7 mM phosphate | | |
| | 230 mM mannitol | | |
| | 10 mM sodium chloride | | |
| 10 | Asp^{B28} insulin 30/70 | 11 | 9 |
| (Comparative) | 7 mM phosphate | | |
| | 174 mM glycerol | | |

## Claims

1. An aqueous insulin preparation comprising:
a) dissolved human insulin analogue and crystals comprising insulin analogue and protamine,
b) 100 to 400 mM of mannitol,
c) 5 to 40 mM of a chloride,
d) a physiologically tolerated buffer, preferably a phosphate buffer,
wherein the concentration of insulin analogue is 60 to 3000 nmol/ml; the weight ratio between dissolved and crystalline insulin analogue is 30:70 to 70:30; the crystals comprises zinc and a phenolic compound; and the analogue of human insulin is one wherein position B28 is Asp, Lys, Leu, Val or Ala and position B29 is Lys or Pro, or des(B28-B30), des(B27) or des(B30) human insulin.

2. An insulin preparation according to claim 1 or 2, wherein the analogue of human insulin is Asp^{B28} human insulin or Lys^{B28}Pro^{B29} human insulin.

## Patentansprüche

1. Wässriges Insulinpräparat, umfassend:
a) gelöstes Humaninsulinanalogon und Insulinanalogon und Protamin umfassende Kristalle,
b) 100 bis 400 mM Mannit,
c) 5 bis 40 mM eines Chlorids,
d) einen physiologisch verträglichen Puffer, vorzugsweise einen Phosphatpuffer,
wobei die Konzentration an Insulinanalogon 60 bis 300 nmol/ml beträgt; das Gewichtsverhältnis zwischen gelöstem und kristallinem Insulinanalogon 30:70 bis 70:30 beträgt; die Kristalle Zink und eine phenolische Verbindung umfassen; und das Humaninsulinanalogon eines ist, in welchem die Position B28 Asp, Lys, Leu, Val oder Ala ist und die Position B29 Lys oder Pro ist, oder des(B28-B30)-, des(B27)- oder des(B30)-Humaninsulin ist.

2. Insulinpräparat nach Anspruch 1, wobei das Humaninsulinanalogon Asp^{B28}-Humaninsulin oder Lys^{B28} Pro^{B29}-Humaninsulin ist.

## Revendications

1. Préparation aqueuse d'insuline comprenant :
a) un analogue d'insuline humaine dissous et des cristaux comprenant un analogue d'insuline et une protamine,
b) du mannitol 100 à 400 mM,
c) un chlorure 5 à 40 mM,
d) un tampon physiologiquement toléré, de préférence un tampon phosphate,
dans laquelle la concentration d'analogue d'insuline est de 60 à 3000 nmol/ml ;
le rapport en poids entre l'analogue d'insuline dissous et celui cristallin est de 30/70 à 70/30 ; les cristaux comprennent du zinc et un composé phénolique ; et l'analogue d'insuline humaine est un analogue dans lequel la position B28 est Asp, Lys, Leu, Val ou Ala et la position B29 est Lys ou Pro, ou l'insuline humaine des (B28-B30), des (B27) ou des (B30).

2. Préparation d'insuline selon la revendication 1 ou 2, dans laquelle l'analogue d'insuline humaine est l'insuline humaine Asp^{B28} ou l'insuline humaine Lys^{B28}_{Pro}^{B29}_{.}
